# EUROPEAN PATENT SPECIFICATION

(11) **EP 1 651 154 B1**
(45) Date of publication and mention of the grant of the patent: **07.02.2007**
(21) Application number: 04756473.7
(22) Date of filing: 30.06.2004
(51) Int. Cl.: A61F 13/15

(54) **COMPRESSION PACKED ABSORBENT ARTICLE**
KOMPRESSIONSVERPACKTER ABSORBIERENDER ARTIKEL
ARTICLE ABSORBANT EMBALLE PAR COMPRESSION

(30) Priority: 17.07.2003 US 622274
(43) Date of publication of application: 03.05.2006
(73) Proprietor: Diaperoos, LLC, Charlotte, NC 28202 (US)
(72) Inventor: KLIPPEN, Michelle, Shakoppee, MN 55379 (US); KLLITZKE, Penny, Darwin, MN 55324 (US)
(74) Representative: Roberts, Gwilym Vaughan
(86) International application number: PCT/US2004/021098
(87) International publication number: WO 2005/009311

(56) References cited:
- EP-A- 0 354 172
- EP-A- 1 400 223
- GB-A- 627 218
- GB-A- 2 366 730
- US-A- 4 594 835
- US-A- 5 986 165
- US-A1- 2002 117 419
- US-B1- 6 475 199

## Description

This application is being filed on 21 June 2004 as a PCT International Patent application in the name of Michelle Klippen, a U.S. citizen, and Penny Klitzke, a U.S. citizen, claiming priority to U.S. Patent Application No. 10/622,274 filed 17 July 2003.

### Technical Field

The present invention generally relates to absorbent articles, and more specifically relates to packaging of a single, unused absorbent article.

### Background

Absorbent articles such as diapers are commonly bulky and inconvenient to carry. As a result, many people must carry extra bags or inconveniently large bags simply to accommodate large absorbent articles. Because these articles are often needed away from the home in sometimes unpredictable situations, they must be transportable and easy to use. Furthermore, absorbent articles should be kept clean while in transport to reduce the chances of transmitting disease or illness, which is a major consideration when dealing with many types of absorbent articles.

Various techniques to package bulky sanitary products such as diapers have been developed. However, none of these techniques provide packaging for bulky, individual absorbent articles that are easy for individual consumers to carry and use one at a time.

For example, one packaging technique that has been developed is vacuum packing a plurality of diapers, which compresses the diapers for ease of shipping bulk product. However, the benefit of the vacuum packing is lost when the package is open and a few diapers are removed to carry in a purse or other bag. Another packaging technique has been vacuum packing a soiled diaper after it is used. Packing soiled diapers provides little or no benefit to the individual consumer who needs to pack and carry bulky items such as diapers or other sanitary, absorbent articles. This shortcoming is especially true because most people immediately dispose of soiled diapers and do not carry them in their purses or diaper bags.

Yet other packaging techniques have been developed for folding sanitary napkins and packaging diapers with wipes and other products. These packaging techniques have limited, if any benefit. Sanitary products tend to be bulky. Folding a product merely changes its dimensions and does not significantly reduce its volume. Additionally, merely placing a wipe in a package with a diaper is convenient, but again, it does not reduce the volume required by the diaper when carrying it in a bag

US-B-6475199 relates to a method of individually packaging an absorbent article. It discloses compressing the absorbent article prior to packaging the absorbent article.

EP-A-0354172 relates to a device for packaging absorbent articles. It discloses compressing absorbent articles whilst they are within containers then sealing a cover strip on to the containers.

### Summary

According to the present invention, there is provided a method and apparatus as claimed in the independent claims.

In general terms, the present invention relates to a sanitary, unused article that is compressed and individually packaged. An individual unused sanitary absorbent article, such as a diaper, may be placed in a compressed state with a small form factor relative to the form factor of the absorbent article in an uncompressed state for convenient handling and transportability of the absorbent article.

One example useful in understanding the claimed invention is a compressed, individually packaged unused absorbent article comprising a sealed package having an internal volume. A single, unused absorbent article is located in the internal volume. The absorbent article has a compressed state and uncompressed state, and the sealed package holding the absorbent article in the compressed state.

Another example comprises a single, unused, sanitary absorbent article having first and second form factors. The second form factor is substantially smaller than the first form factor. A sealable package has an internal volume sized to receive the absorbent article when in the first form factor. The sealable package is configured to be sealed with the absorbent article maintained in second form factor inside the package when sealed.

Another example comprises a sealed package formed with an airtight material. A folded diaper has compressed and uncompressed states, and the folded diaper when in compressed state occupies about 30% to about 70% less volume than when in the uncompressed state. The package maintains the absorbent article in the compressed state. A zip strip is formed in the package allowing easy opening of the package and access to the absorbent article.

### Description of the Drawings

FIGURE 1 is a perspective view of a compression packaged absorbent article according to principles of the invention

FIGURE 2 is a side view of the compression packaged absorbent article shown in FIG. 1.

FIGURE 3 is a side view of one example folding arrangement for an absorbent article using two folding axes.

FIGURE 4 is a side view of another example folding arrangement for an absorbent article using two folding axes.

FIGURE 5 is a side view of a rolled absorbent article.

FIGURE 6 is a side view of yet another example folding arrangement for an absorbent article using three folding axes.

FIGURE 7 is a exploded perspective view of the components of the vacuum packaged absorbent article shown in FIG. 1.

FIGURE 8 is a cross-sectional side view of the compression packaged absorbent article shown in FIG. 1 before the absorbent article has been compressed.

FIGURE 9 is a perspective view of an example compression packaged diaper kit that includes a diaper, a wet wipe, and a changing pad according to principles of the invention.

### Detailed Description

Various embodiments of the present invention will be described in detail with reference to the drawings, wherein like reference numerals represent like parts and assemblies throughout the several views. Reference to various embodiments does not limit the scope of the invention, which is limited only by the scope of the claims attached hereto. Additionally, any examples set forth in this specification are not intended to be limiting and merely set forth some of the many possible embodiments for the claimed invention. There are alternative embodiments for all of the structures and methods disclosed herein regardless of whether specific alternatives are set forth.

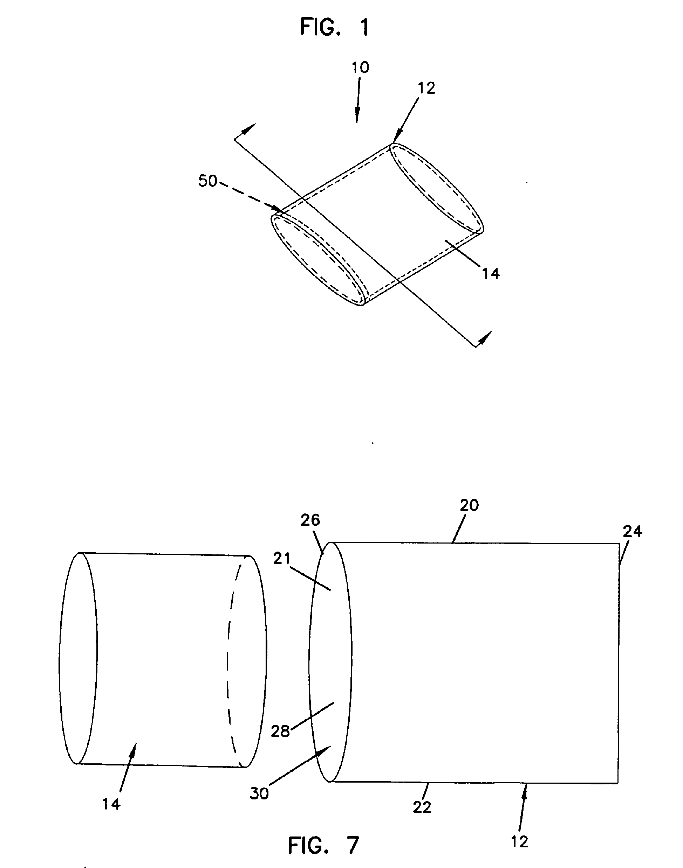

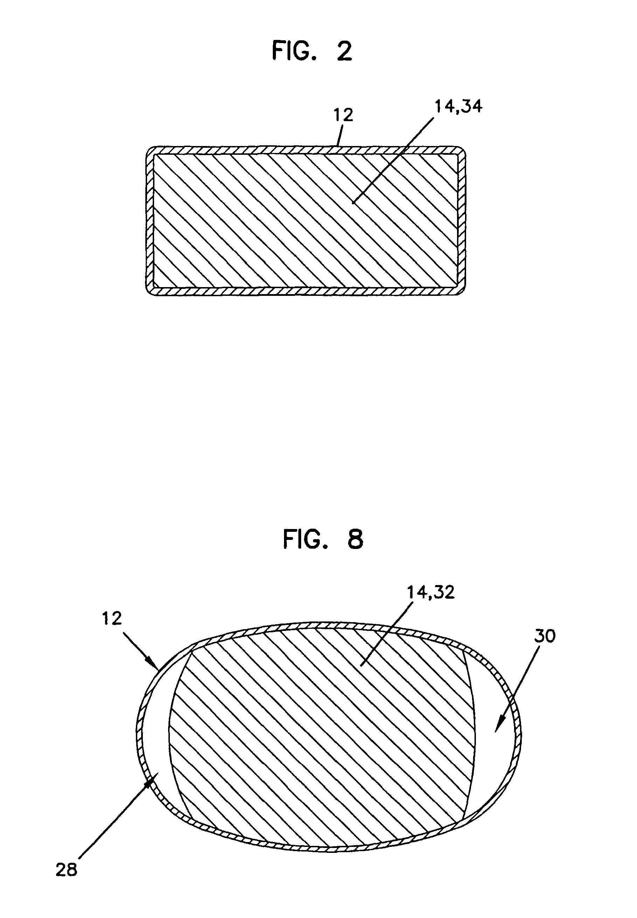

Referring to Figures 1 and 2, a packaging material **12** includes first and second opposing side portions **20** and **22** and has first and second ends **24** and **26.** The packaging material defines an internal volume **30.** A single, unused absorbent article is positioned within the internal volume **30** of the packaging material **12.** In one possible embodiment, the packaging material **12** is sealed so that internal volume **30** is entirely enclosed or completely surrounded and isolated from the area external to the packaging materials.

The packaging material **12** can be formed with a variety of different configurations. In at least some embodiments, for example, the first and second side portions **20** and **22** are formed from separate flat panels. In yet other possible embodiments, the first and second side portions **20** and **22** are formed from a single, tubular sheet of material having an endless wall.

The packaging material **12** can be formed with a variety of different material. In one possible embodiment, the packaging material **12** is formed with a material that is substantially impermeable to water. An advantage of this embodiment is that it protects the absorbent article if the package is inadvertently exposed to moisture. In another possible embodiment, the material used to form the packaging material **12** is substantially impermeable to air. This embodiment enables a vacuum to be drawn and maintained in the interior volume thereby allowing the first and second side portions **20** and **22** of the packaging material **12** to collapse and compress the single, unused absorbent article **14.**

Additionally, the packaging material **12** includes a selectively openable region **50** that allows a user to easily open the packaging material **12** and access the single, unused absorbent article **14.** The selectively openable region **50** can have a variety of different structures. Examples include a score, perforation line, pull strip, resealable zip strip, or other structure that facilitates easy opening of the packaging material **12** for access to the internal volume **30** of the packaging **12.** In at least some possible embodiments, the openable region **50** does not compromise the sealed or resealable nature of the packaging material **12,** which allows a vacuum to be drawn in the interior volume and also prevents moisture from entering the interior volume **30.** Additionally, in at least some possible embodiments, the openable region **50** is resealable, which may be advantageous, for example, to repackage the absorbent article **14** after use of the article.

The openable region **50** may be positioned on either of the side portions **20** and **22** of the package material or on either end **24** or **26** of the packaging material **12.** Additionally, the openable region **50** can have any orientation. It may run the width or the length of the package. Additionally, the openable region may have any length. It may extend for the entire length or the packaging material, the entire width of the packaging material, or any portion thereof. Additionally, the openable region may extend entirely around the packaging material and form an endless line.

The single, unused absorbent article **14** has first and second states **32** and **34.** The first state **32** is an uncompressed state in which the single, unused absorbent article has its natural form factor and can be used for its intended purpose.
The second state **34** is a compressed state in which the form factor or overall volume of the single, unused absorbent article **14** is reduced so that it can be carried and stored while taking up minimal storage space. The packaging material **12** holds the single, unused absorbent article **14** in the second state until a user selectively removes the single, unused absorbent article **14** from the internal volume **30.**

Examples of the articles that can form the single, unused absorbent article 14 include children's and adult diapers, sanitary napkins and other feminine hygiene products, first aid and other medical products, or any other absorbent article that a consumer might want to carry. The term diaper is used herein for ease of illustration and description.

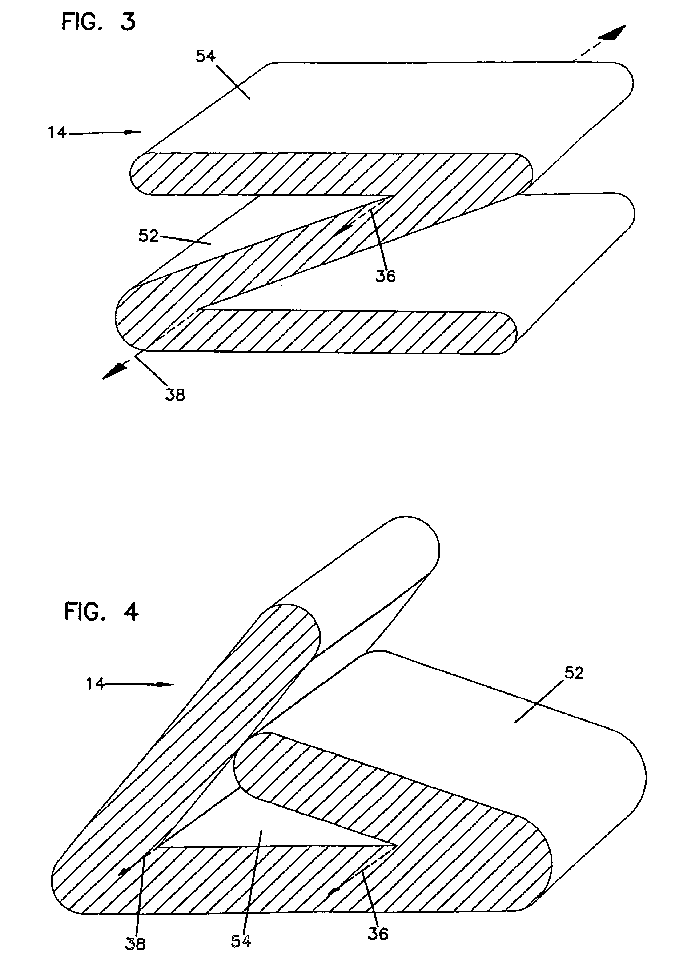

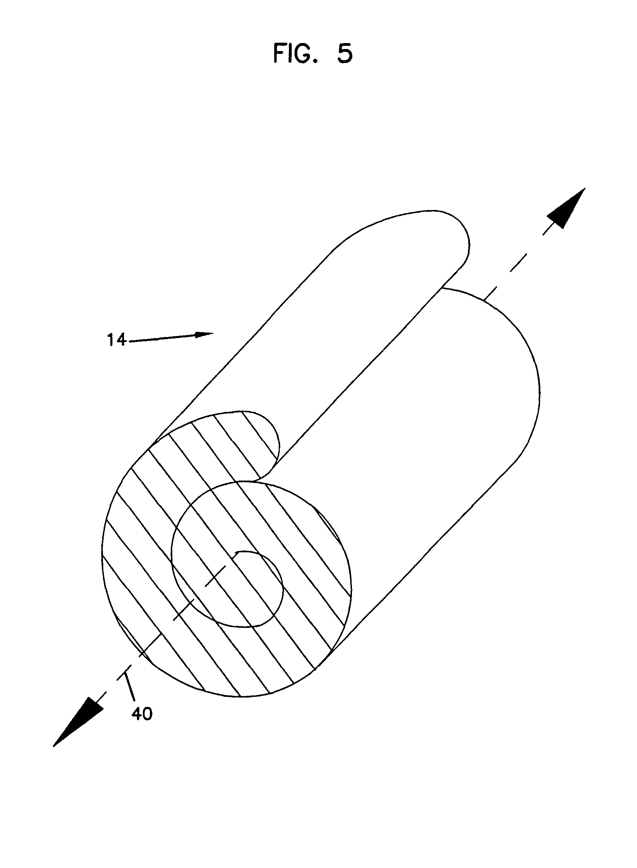

Additionally, the single unused diaper **14** is folded when positioned in the interior volume. Referring to Figure 3, for example, one possible folding arrangement folds the single, unused diaper **14** about two fold axes **36,38**. The diaper includes first and second primary surfaces **52,54** that are folded over each other along the fold axes **36,38** to form a generally"Z"shaped folding arrangement.
Referring to Figure 4, another possible folding arrangement involves folding the single, unused diaper about two fold axes **36,38.** In this configuration, the primary surfaces **52,54** are folded about fold axes **36,38** to form a generally "C" shaped folding arrangement. Figure 5 illustrates yet another folding arrangement for the single, unused diaper **14** in which the diaper **14** is rolled about a single axis **40.**

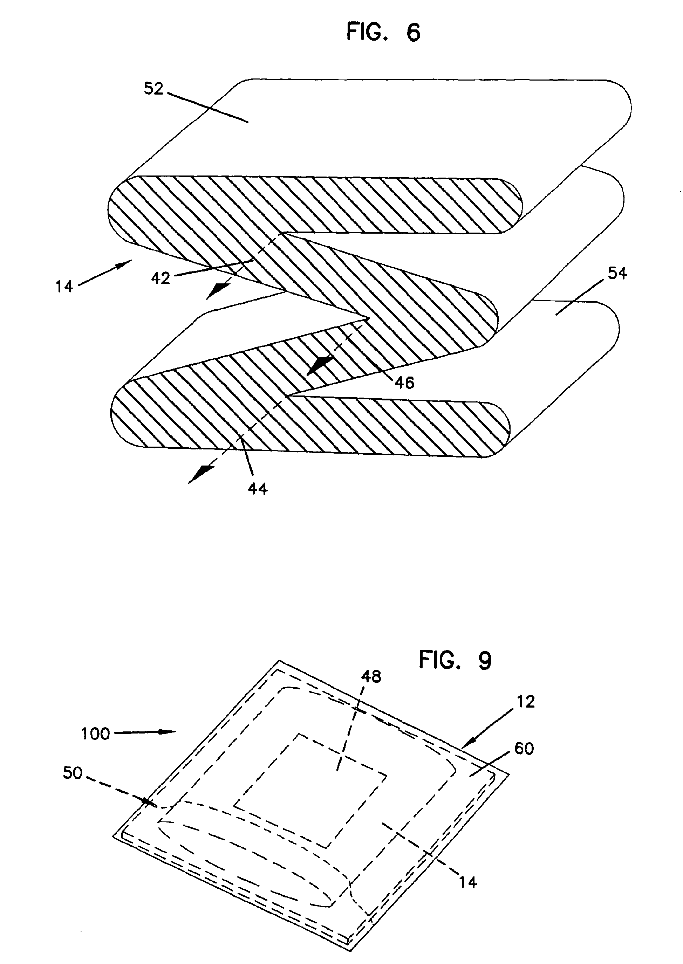

Figure 6 illustrates a folding arrangement in which the single, unused diaper is folded about three fold axes **42,44,** and **46.** In this arrangement, the primary surfaces **52,54** are folded over each other twice to form a generally "M" shaped folding arrangement.

Referring to Figures 7 and 8, the single, unused absorbent diaper **14** and the packaging material **12** can be assembled in a variety of different ways. In at least some possible embodiment, the single, unused absorbent diaper **14** and the packaging material **12** are assembled using a vacuum. In these embodiments, the packaging material **12** forms an opening **28,** and the internal volume **30** is sized to receive the single, unused absorbent diaper **14** when it is folded, but still in the first, uncompressed state **32.** In this embodiment, the interior volume is slightly larger than the single, unused absorbent diaper **14** when it is in a state (e. g. , folded, semi- compressed, or fully compressed) ready to be inserted into the packaging material.
The single, unused absorbent diaper **14** is folded and then inserted into the interior volume **30,** A vacuum is then drawn in the interior volume **30,** which causes the side portions **20** and **22** to collapse toward one another and compress the single, unused sanitary diaper **14** into a smaller form factor so that it occupies a smaller volume. The opening **28** is then sealed to maintain the vacuum in the interior volume **30.**

There are many other possible ways to assemble the single, unused absorbent diaper **14** and the packaging material **12.** The single, unused absorbent diaper **14** can be compressed into the second state **34** and then is inserted into the interior volume **30.** The single, unused absorbent diaper **14** is allowed to expand to the dimensions of the interior volume **30** as defined by the first and second opposing side portions **20** and **22.** In this embodiment, a vacuum is not used to place the single, unused absorbent diaper **14** into the second, compressed state **34.**
Additionally, in this embodiment, the packaging material **12** may not be sealed. For example, the first and second ends **24** and **26** can be open so that the packaging material **12** is generally tubular in configuration.

In another possible embodiment, the packaging material **12** is formed with a heat shrink material. In this embodiment, the single, unused absorbent diaper 14 is inserted into the interior volume 30 and then the packaging material 12 is heated causing it to shrink and compress the single, unused absorbent diaper 14 into the second state **34.** Again, in this embodiment, the packaging material 12 may not be sealed.

Additionally, in other embodiments, these basic assembly techniques can be combined. For example, assembly may include the actions of compressing the single, unused absorbent diaper **14** into a compressed or semi-compressed state, inserting the compressed or semi-compressed single, unused absorbent diaper **14** into the interior volume **30,** and then drawing a vacuum. Many other embodiments and combinations of actions are possible while assembling the single, unused absorbent diaper **14** and the packaging material **12.**

As demonstrated in the following examples, the packaging of a single, unused diaper 14 as described results in a substantial reduction in the diaper's form factor or volume. In one possible embodiment, for example, the volume from the first, uncompressed state 32 to the second, compressed state 34 is reduced by about 40% or more. The following examples set forth the experimental results of packaging various sizes and brands of diapers with a compressive vacuum pressure of 0.035 megapascals (MPa). Although certain brands and sizes were tested at this pressure, it is understood that the packaging techniques and structure disclosed herein can be used with any size and type of single absorbent article with any type and amount of compressive force sufficient to compress the single absorbent article and reduce its volume.

### Example 1

A size 2 children's Pampers diaper has a length of 18.5cm (7.3 inches), a width of 10.9cm (4.3 inches), and a height of 1.8cm (0.7 inches) in an uncompressed state (first form factor), and a length of 11.2cm (4.4 inches), width of 7.6cm (3.0 inches), and height of 2cm (0.8 inches) in a compressed state (second form factor). As a result, the first form factor has a volume of about 373.6cm³ (22.8 in³) and the second form factor has a volume of about 165.5cm (10.1 in³), which is about a 55.54% reduction in volume from the first to the second form factor.

### Example 2

A size 5 children's Huggies diaper has a length of 24.13cm (9.5 inches), a width of 10.9cm (4.3 inches), and a height of 1.8cm (0.7 inches) in an uncompressed state (first form factor),
and a length of 7.4cm (2.9 inches), width of 10.9cm (4.3 inches), and height of 2.3cm (0.9 inches) in a compressed state (second form factor). As a result, the first form factor has a volume of about 481.8cm³ (29.4 in³) and the second form factor has a volume of about 181.9cm³ (11.1 in³), which is about a 62.29% reduction in volume from the first to the second form factor.

### Example 3

An adult extra-large Depends diaper has a length of 21.8cm (8.6 inches), width of 14.5cm (5.7 inches) and height of 4.1cm (1.6 inches) in an uncompressed state (first form factor), and a length of 13.2cm (5.2 inches), width of 10.4cm (4.1 inches), and height of 3cm (1.2 inches) in a compressed state (second form factor). As a result, the first form factor has a volume of about 1270cm³ (77.5 in³) and the second form factor has a volume of about (25.5 in³), which is about a 67.15% reduction in volume from the first to the second form factor.

### Example 4

An adult medium-sized Depends diaper has a length of 24.1cm (9.5 inches), width of 12.7cm (5 inches) and height of 2.2cm (0.88 inches) in an uncompressed state (first form factor), and a length of 10.8cm (4.24 inches), width of 6.9cm (2.72 inches), and height of 3.8cm (1.5 inches) in a compressed state (second form factor). As a result, the first form factor has a volume of about 685cm³ (41.8 in³) and the second form factor has a volume of about 283.5cm (17.3 in³), which is about a 58.61% reduction in volume from the first to the second form factor.

Other examples have changes in volume between the first and second form factors of greater than 30%, with changes in volume of about 80% to 90% being possible in some cases of highly porous absorbent materials, such as cotton balls and sponges. The percentage of reduced volume is typically dependent on the article being compressed. Thus, in one possible embodiment, the change in volume between first and second form factors is in the range from about 30% to about 70%.
In another embodiment the change in volume is about 30% or more. Another embodiment has a change in volume of about 40% or more. Another embodiment has a change in volume of about 55% or more.

Referring to Figure 9, at least some possible alternative embodiments may include kits having an item complimentary to the single, unused absorbent article. For example, the kit 100 may include one or more wipes **48** positioned in the interior volume together with a single, unused diaper **14**. In alternative embodiments, the wipes **48** may be individually wrapped or may be located between moisture impermeable surfaces of the diaper **14.** Other products may also be added to the package, such as, for example, a bag 60 for disposal of the diaper after it is used, packaged lotion or ointments, and deodorizers. In other embodiments, some of the complementary items held in the packaging 12 are compressed, while other items are uncompressed when the packaging material 12 is sealed.

The various embodiments described above arc provided by way of illustration only and should not be construed to limit the invention. Those skilled in the art will readily recognize various modifications and changes that may be made to the present invention without following the example embodiments and applications illustrated and described herein.

## Claims

1. A method of compressing and packaging a single unused sanitary absorbent article (14) in a sealable, compressible package (12), the method comprising:
compressing a single unused sanitary absorbent article (14);
inserting the single unused sanitary absorbent article (14), while compressed, into a package (12):
drawing a vacuum within the package (12); and
sealing the package (12), the sealed package maintaining the article (14) in a compressed state.

2. A method as claimed in claim 1 wherein the absorbent article (14) is:
a diaper;
a compressible feminine hygiene product or
a medical product.

3. A method as claimed in claim 1 wherein, before compressing the absorbent article (14), the absorbent article (14) is:
folded together about two fold axes (36, 38):
folded together about three fold axes (42, 44, 46) or
rolled.

## Patentansprüche

1. Verfahren zum Komprimieren und Verpacken eines einzelnen unbenutzten absorbierenden Sanitärartikel (14) in einer versiegelbaren, komprimierbaren Verpackung (12), wobei das Verfahren aufweist:
Komprimieren eines einzelnen unbenutzten absorbierenden Sanitärartikels (14)
Einführen des einzelnen unbenutzten absorbierenden Sanitärartikels (14) im komprimierten Zustand in eine Packung (12);
Erzeugen eines Vakuums innerhalb der Packung (12); und
Versiegeln der Packung (12), wobei die versiegelte Packung den Artikel (14) in einem komprimierten Zustand hält.

2. Verfahren nach Anspruch 1, wobei der absorbierende Artikel (14) ist:
eine Windel;
ein komprimierbares feminines Hygieneprodukt oder
ein medizinisches Produkt.

3. Verfahren nach Anspruch 1 wobei, vor dem Komprimieren des absorbierenden Artikels (14), der absorbierende Artikel ist:
um zwei Faltungsachsen (36, 38) zusammengefaltet;
um drei Faltungsachsen (42, 44, 46) zusammengefalten oder
gerollt.

## Revendications

1. Procédé de compression et d'emballage d'un unique article absorbant sanitaire inutilisé (14) dans un emballage compressible (12) pouvant être fermé hermétiquement, le procédé comprenant :
la compression d'un unique article absorbant sanitaire inutilisé (14);
l'insertion de l'unique article absorbant sanitaire inutilisé (14), tandis qu'il est comprimé, dans un emballage (12);
la formation d'un vide à l'intérieur de l'emballage (12); et
la fermeture hermétique de l'emballage (12), l'emballage fermé hermétiquement maintenant l'article (14) à l'état comprimé.

2. Procédé selon la revendication 1, dans lequel l'article absorbant (14) est :
une couche;
un produit d'hygiène féminine compressible ou
un produit médical.

3. Procédé selon la revendication 1, dans lequel, avant compression de l'article absorbant (14), l'article absorbant (14) est :
replié autour de deux axes de pliage (36, 38);
replié autour de trois axes de pliage (42, 44, 46) ou
enroulé.
